# EUROPEAN PATENT APPLICATION

(11) **EP 2 374 809 A1**
(43) Date of publication of application: **12.10.2011**
(21) Application number: 10461509.1
(22) Date of filing: 16.03.2010
(51) Int. Cl.: C07H 1/00, C07H 5/06

(54) **A process for the preparation of 6-O-acetyl-2-azido-3,4-di-O-benzyl-2-deoxy-D-glucopyranose**

(71) Applicant: Adamed sp. z o.o., 05-152 Czosnów k/Warszawy (PL); Zaklad Doswiadczalny CHEMIPAN Instytutu Chemii Fizyczneh i Instytutu Chemii Organicznej Polskiej Akademii Nauk, 01-224 Warszawa (PL)
(72) Inventor: Majka, Zbigniew, 39-102, Lubzina (PL); Raczko, Jerzy, 01-957, Warszawa (PL); Bartoszewski, Zbigniew,, 02-352, Warszawa (PL); Powala, Sylwester, 05-126, Nieporet (PL); Radwanski, Bogdan, 05-800, Pruszków (PL)
(74) Representative: Sitkowska, Jadwiga

(57) **Abstract**

The invention relates to a process for the preparation of 6-O-acetyl-2-azido-3,4-di-O-benzyl-2-deoxy-D-glucopyranose, which process comprises reaction of 1,6-anhydro-2-azido-2-deoxy-D-glucopyranose with benzyl bromide in acetonitrile in the presence of solid sodium hydroxide to form 1,6-anhydro-2-azido-3,4-di-O-benzyl-2-deoxy-D-glucopyranose; subsequent reaction of 1,6-anhydro-2-azido-3,4-di-O-benzyl-2-deoxy-D-glucopyranose with acetic anhydride in the presence of trifluoroacetic acid to form 1,6-di-O-acetyl-2-azido-3,4-di-O-benzyl-2-deoxy-D-glucopyranose; and treatment of 1,6-di-O-acetyl-2-azido-3,4-di-O-benzyl-2-deoxy-D-glucopyranose with piperidine in THF.

6-0-Acetyl-2-azido-3,4-di-O-benzyl-2-deoxy-D-glucopyranose is useful as a building block for synthesis of glucosamine oligosaccharides.

## Description

The invention relates to the process for the preparation of 6-O-acetyl-2-azido-3,4-di-O-benzyl-2-deoxy-D-glucopyranose, useful as an intermediate for the synthesis of oligosaccharides.

6-O-acetyl-2-azido-3,4-di-O-benzyl-2-deoxy-D-glucopyranose is a known compound that finds its use in the carbohydrates chemistry, such as a precursor of active pharmaceutical substances, in particular oligosaccharides and sulphonated oligosaccharides containing D-glucosamine units, such as for example sulphonated pentasaccharide fondaparinux.

The use of 6-O-acetyl-2-azido-3,4-di-O-benzyl-2-deoxy-D-glucopyranose as a glucosyl donor - monomeric building block for the synthesis of sulphonated oligosaccharides, including pentasaccharide fondaparinux, is described by J.D.C. Codée et al in J. Am. Chem. Soc. 2005, 127, 3767-3773. The process outlined there is presented in the scheme below.

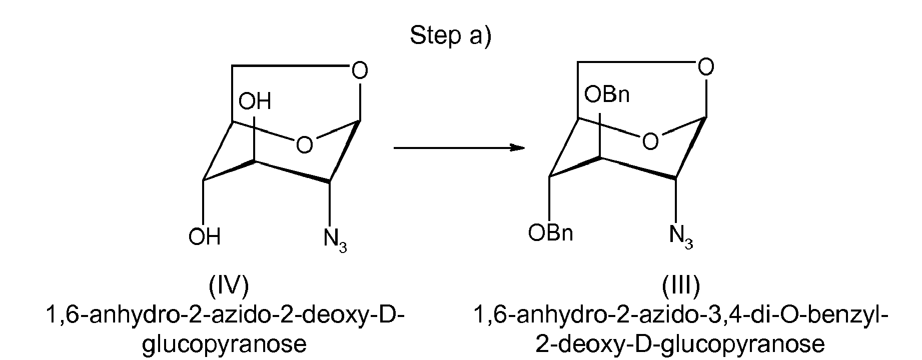

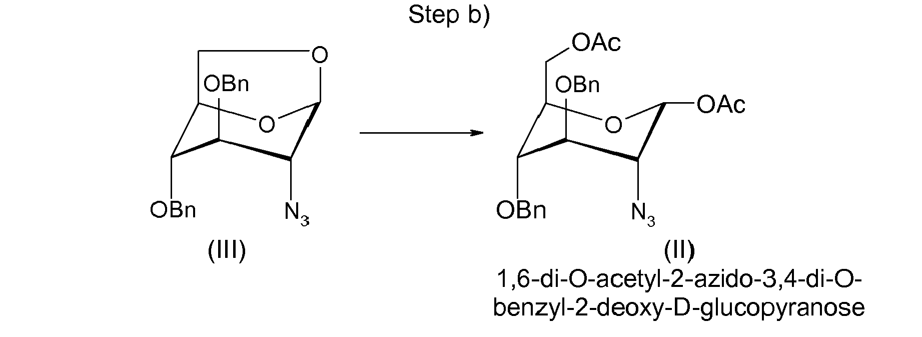

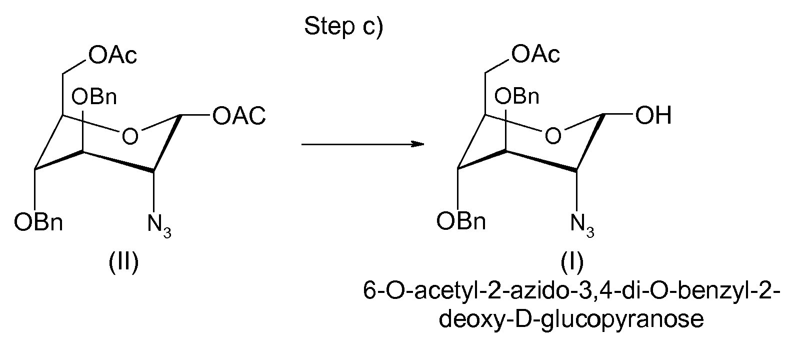

According to this process, 6-O-acetyl-2-azido-3,4-di-O-benzyl-2-deoxy-D-glucopyranose (I) can be obtained from 1,6-anhydro-2-azido-2-deoxy-D-glucopyranose (IV) in three steps by a) protection of hydroxyl groups at positions 3 and 4 using benzylation with benzyl bromide in the presence of sodium hydride in dimethylformamide (with declared yield of 89%), followed by the second step of b) acetylation in positions 1 and 6 with acetic anhydride in tetrahydrofuran, and the third step of c) regioselective removal of the acetyl protective group in position 1 using 6% piperidine in tetrahydrofuran, with a declared total yield of the second and third steps 94%. Neither details of carrying out these three steps, nor the process of preparation of the final product (I) are disclosed.

Benzylation of 1,6-anhydro-2-azido-3,4-di-O-benzyl-2-deoxy-D-glucopyranose (IV) with benzyl bromide in dimethylformamide in the presence of sodium hydride as an alkaline condensing agent is described in more detail by J. Hawley et al in Eur.J.Org.Chem., 2002, 1925 - 1936. The disadvantages of using sodium hydride are its high price, inflammability and liberation of large amounts of inflammable hydrogen while contacted with water, and as a consequence, the necessity of keeping strictly anhydrous reaction medium.

Benzylation of 1,6-anhydro-2-azido-3,4-di-O-benzyl-2-deoxy-D-glucopyranose (IV) with benzyl bromide in the presence of barium hydroxide in dimethylformamide, with declared yield of 97%, is described by D.Tailler et al in J.Chem.Soc. Perkin Trans 1, 1992, 3163-3164. The same reaction performed in the presence of a mixture of barium oxide and barium hydroxide in DMF is described e.g. by F.Dasgupta, Per J.Garegg, in Synthesis 1988, 626-628. These prior art processes using barium hydroxide are in turn inconvenient because of the necessity of removing from the reaction medium very toxic barium salts that form extremely fine precipitate requiring long filtration, as well as high prices of barium compounds. In addition, it appeared that in spite of long reaction times it is not possible to achieve high yields declared in the prior art.

Additionally, dimethylformamide used as a solvent in benzylation reaction in prior art processes is difficult to evaporate. The regeneration of dimethylformamide from aqueous solutions after work-up of reaction mixture is also difficult.

The known processes of benzylation with benzyl bromide conducted as in the above step a) proved useful only in a lab scale and scaling up was associated with technical problems.

Acetylation of 1,6-anhydro-2-azido-3,4-di-O-benzyl-2-deoxy-D-glucopyranose (III) to 1,6-di-O-acetyl-2-azido-3,4-di-O-benzyl-2-deoxy-D-glucopyranose (II) in the presence of catalytical amounts of trifluoroacetic acid as in the above step b) is described by H. Paulsen i M. Schuller in Liebigs Ann. Chem 1987, 249. This method yields the mixture of both anomer alpha and beta. The mixture of both anomers is also obtained by acetylation with acetic anhydride in the presence of sulphuric acid in nitromethane using the process described by H. Paulsen and W. Stenzel in Chem. Ber., 1978,111,2334-2347.

In the third step of the known process, acetyl group in position 1 of 1,6-di-O-acetyl-2-azido-2-deoxy-3,4-di-O-benzyl-D-glucopyranose is regioselectively deacetylated with piperidine in tetrahydrofuran, to form 6-0-acetyl-2-azido-3,4-di-O-benzyl-2-deoxy-D-glucopyranose.

The use of this regioselective deacetylation for obtaining 6-0-acetyl-2-azido-3,4-di-O-benzyl-2-deoxy-D-glucopyranose as an intermediate, which without isolation is further converted by treating with trichloroacetonitrile to a corresponding trichloroacetimidate derivative, is described by R. Verduyn et al in Recl. Trav. Chim. Pay-Bas, 1990, 109, 591-593; N.M. Spijker, J-W. Slief, and C.A.A. van Boeckel in J.Carbohydrate Chem., 1993, 1017 - 1041. The problem during this selective deacetylation is partial epimerization in the position 2, at the azide-substituted carbon atom, resulting in formation of a corresponding mannopyranose derivative by-product as an impurity. In the known process glucopyranose and mannopyranose trichloroacetamidate derivatives were separated by silica gel chromatography.

The present invention solves a problem of providing a process for the preparation of 6-O-acetyl-2-azido-3,4-di-O-benzyl-2-deoxy-D-glucopyranose capable for scaling up to a technical scale, without using inconvenient bases and solvents in the benzylation step.

The problem solved by the present invention is also to provide a process for the preparation of 6-O-acetyl-2-azido-3,4-di-O-benzyl-2-deoxy-D-glucopyranose in a crystalline form devoid from by-products without using chromatographic methods of separation and purification, inconvenient at technical scale.

The process for the preparation of 6-O-acetyl-2-azido-3,4-di-O-benzyl-2-deoxy-D-glucopyranose according to the present invention comprises the following steps:
a) reaction of 1,6-anhydro-2-azido-2-deoxy-D-glucopiranose with benzyl bromide in acetonitrile in the presence of solid sodium hydroxide to obtain 1,6-anhydro-2-azido-3,4-di-O-benzyl-2-deoxy-D-glucopyranose;
b) reaction of 1,6-anhydro-2-azido-3,4-di-O-benzyl-2-deoxy-D-glucopyranose with acetic anhydride in the presence of trifluoroacetic acid to obtain 1,6-di-O-acetyl-2-azido-3,4-di-O-benzyl-2-deoxy-D-glucopyranose; and
c) treating 1,6-di-O-acetyl-2-azido-3,4-di-O-benzyl-2-deoxy-D-glucopyranose with pyridine in THF, to obtain 6-O-acetyl-2-azido-3,4-di-O-benzyl-2-deoxy-D-glucopyranose.

It has been unexpectedly found that benzylation of 1,6-anhydro-2-azido-2-deoxy-D-glucopyranose in step a) can be performed in heterophase system, using solid sodium hydroxide as a base and acetonitrile as reaction solvent. This was surprising because of belief prevailing in the prior art concerning instability of sugars in the presence of sodium hydroxide.

In step a) anhydrous reaction conditions are not necessary, therefore there is no need of dehydration of the solvent.

Acetonitrile solvent after regeneration is almost completely recovered and recycled to the process, thus avoiding formation of toxic sewages.

To isolate the product of step a) the reaction mixture can be subjected to standard work-up, consisting of filtering off inorganic residues (sodium bromide, unreacted sodium hydroxide), and distilling off acetonitrile under reduced pressure, followed by crystallization from n-hexane. In this manner, the product of step a) is obtained after relatively short reaction time with very good yield and purity.

The reaction in step b) is performed in the presence of catalytic amounts of trifluoroacetic acid, at increased temperature and with stirring. Temperature is maintained at approximately 50°C, preferably in the range of 48-53°C. After completion of the reaction, non-reacted excess of acetic anhydride is distilled off under reduced pressure, the residue is treated with toluene and the solvent is distilled off again. Operations of addition and evaporation of toluene can be repeated several times. The product of step b) is in the form of mixture of two anomers a i f3 and can be used in the next step as it is, without additional purification.

The reaction in step c) is performed with stirring at room temperature. Heating is not required.

According to the process of the invention, the mixture of anomers a i f3 of the product (I) is obtained in the reaction in step c).

After completion of the reaction, tetrahydrofuran can be removed by distilling off under reduced pressure and the residue is subjected to work-up for piperidine removal. For this purpose, the residue is dissolved in organic solvent, such as for example ethyl acetate, and washed several times with diluted aqueous acid solution, e.g. hydrochloric acid, and then several times with water. Subsequently, after drying, the solvent is evaporated to give a crude product of step c).

The crude product of step c) can be subjected to work-up procedure consisting of dissolving in isopropanol, adding n-hexane to precipitate crystalline product of step (c), and filtration of the crystalline precipitate.

Preferably, the filtrate from filtration of the above first crop of crystalline precipitate, after evaporation of organic solvents, can be subjected to work-up with a mixture of isopropanol and n-hexane to obtain a second crop of crystalline precipitate of the product of step (c), and subsequent filtration of the second crop. The crops of crystalline precipitates are then combined.

The filtrate form the filtration of the second crop can be also subjected to the same work-up as above by evaporation and treating with the mixture of isopropanol and n-hexane, to obtain further crops of the product from step c). Preferably, the residue after evaporation of the filtrate before further treatment with the mixture of isopropanol and n-hexane can be purified by dissolving in an organic solvent, such as methylene dichloride, treating with an adsorbent, such as silica gel, to remove colour impurities, and evaporation of this solvent.

Crystalline precipitate of 6-O-acetyl-2-azido-3,4-di-O-benzyl-2-deoxy-D-glucopyranose thus obtained has the purity of 99% and the impurity, 6-0-acetyl-2-azido-3,4-di-O-benzyl-2-deoxy-D-mannopyranose, remains in mother liquors.

Furthermore, after recrystallization in the isopropanol/hexane solvent system described above, desired compound is obtained in the form of pure anomer α, this being an additional advantage if this compound is used as a starting material for a synthesis when starting configuration of glucosyl donor is of importance.

### Example

### 6-O-Acetyl-2-azido-3,4-di-O-benzyl-2-deoxy-D-glucopyranose

### a) 1,6-Anhydro-2-azido-3,4-di-O-benzyl-2-deoxy-D-glucopyranose.

The reactor is charged with 1.03 kg (5,5 mol) of 1,6-anhydro-2-azido-2-deoxy-D-glucopyranose and 13 dm³ of acetonitrile, then 0,5 kg (12,5 moles) of solid NaOH is added. The solids do not dissolve. The mixture is heated with heating mantle to 23°C, then the heating is turned off (the solids are still not dissolved). While maintaining the mixture at this temperature, 1.92 kg (11.2 moles) of benzyl bromide is added at once with intense stirring. Within 4 hours of stirring the temperature increases to 32.5°C and then begin to decrease. (A separate experiment has shown that conducting the reaction at temperatures below 30°C doesn't give satisfactory results). The mixture is stirred for a total of 8 hours, then left overnight. The reaction mixture is filtered off from the sticky precipitate of sodium bromide and unreacted sodium hydroxide, evaporated under reduced pressure at a bath temperature of 50°C and, while stirring, poured into reactor containing 2 dm³ of hexane. The whole is cooled to 0-5°C. If the precipitation does not occur within 1 hour, hexane should be decanted from above the oily residue and new portion of hexane added, and cooling with stirring continued until precipitation. The solid precipitate is filtered off, washed with 1 dm³ of cold hexane and dried at 40°C. Off-white, dry title product (1680 g) is obtained with 98% purity (HPLC), m.p. 67-68°C (hexane) and 85% yield.
¹H NMR (400 MHz, CDCl₃) δ (ppm): 7.36-7.30 (m, 10 H), 5.49 (s, 1H), 4.64-4.61 (m, 1H), 4.61 (d, 1H, *J* = 12.4 Hz), 4.58 (d, 1H, *J* = 12.4 Hz), 4.54 (d, 1H, *J* = 11.9 Hz), 4.50 (d, 1H, *J* = 11.9 Hz), 4.01 (dd, 1H, *J_{3,4}*= 7.3 Hz, *J_{4,5}=* 0.9 Hz), 3.72 (dd, 1H, *J_{3,4}*= 7.3 Hz, *J_{2,3}*= 5.9 Hz), 3.66-3.64 (m, 1H), 3.39-3.36 (m, 1H), 2.29-3.26 (m, 1H).
¹³C NMR (100 MHz, CDCl₃) δ (ppm):137.34, 137.19, 128.54, 128.53, 128.05, 127.99, 127.88, 127.77, 100.49, 76.12, 75.78, 74.35, 72.32, 71.30, 65.29, 59.82.

### b) 1,6-di-O-Acetyl-2-azido-3,4-di-O-benzyl-2-deoxy-D-glucopyranose.

The reactor is charged with 1650 g of 1,6-anhydro-2-azido-3,4-di-O-benzyl-2-deoxy-D-glucopyranose obtained as described in the above step a) and 6.76 kg of acetic anhydride. Subsequently, 740 g of trifluoroacetic acid is added. The temperature increases from 18 to 22°C. The whole is heated to 50°C and kept at this temperature for 6 hours. On the next day the reaction mixture is concentrated in evaporator, 3 dm³ of toluene is then added to the residue and the mixture is evaporated at the bath temperature 60°C, until condensate ceased to appear in a condenser. This operation is repeated two more times using 1.5 dm³ of toluene each time. The product is cooled down and weighed. The title product (2.2 kg) in the form of a solid mass is obtained with a purity of 92% (HPLC), m.p. 94-100°C (hexane-ethyl acetate 4:1). Yield calculated on a pure product is 96%.
¹H NMR (400 MHz, CDCl₃) δ (ppm): 7.41-7.25 (m, 10H), 6.23 (d, H, *J*= 3.5 Hz), 4.94 (d, 1H, *J* = 10.6Hz), 4.90 (d, 1H, *J* = 10.6 Hz), 4.87 (d, 1H, *J* = 10.8 Hz), 4.59 (d, 1H, *J* = 10.8 Hz), 4.26 (brd, 2H, *J* = 3.1 Hz), 3.97 (dd, 1H, *J₃,₄*= 9.0 Hz, *J_{4,5}*= 10.2 Hz), 3.93 (dt, 1H, *Jₜ=* 3.1 Hz, *J_{4,5}=* 10.2), 3.63 (dd, 1H, *J_{3,4}*= 9.0 Hz, *J_{2,3}*= 10.2 Hz), 3.60 (dd, 1H, *J_{1,2}*= 3.5 Hz, *J_{2,3}*= 10.2 Hz), 2.16 (s, 3H), 2.03 (s, 3H).
¹³C NMR (100 MHz,CDCl₃), δ (ppm): 170.54, 168.76, 137.43, 137.20, 128.62, 128.58, 128.22, 128,13, 128.07, 90.37, 80.54, 77.21, 75.69, 75.30, 71.28, 62.73, 62.31, 20.93, 20.77.

### c) 6-O-Acetyl-2-azido-3,4-di-O-benzyl-2-deoxy-D-glucopyranose.

The reactor is charged with 2.2 kg of 1,6-di-O-acetyl-2-azido-3,4-di-O-benzyl-2-deoxy-D-glucopyranose obtained in the preceding step b) and 9.7 dm³ of THF. Subsequently 1.3 dm³ of piperidine is added. Within 3 hours the temperature increases from 16 to 22°C and then slowly begin to decrease. After total 20 hours of stirring at ambient temperature (16°C) TLC analysis (toluene-ethyl acetate, 5:1) confirms the lack of the starting material in the reaction mixture. The mixture is evaporated at a bath temperature of 35°C to yield 2.9 kg of sticky residue, which is dissolved in 15 dm³ of ethyl acetate in an extractor and washed with 2 x 12 dm³ of 4% hydrochloric acid and then with 2 x 12 dm³ of water. The organic layer is dried over 500 g of sodium sulphate, then the drying agent is filtered off and the filtrate is evaporated. The crystallizing residue is transferred to trays and air-dried, to yield 1680 g of crude title product.

### d) Purification of 6-O-acetyl-2-azido-3,4-di-O-benzyl-2-deoxy-D-glucopyranose

To the evaporator flask 1680 g of crude product from step c) and 840 g of isopropanol are added and stirred in the evaporator without vacuum at 60°C bath until dissolution. The mixture is transferred to the tank and, after addition of 840 g of hexane, left in refrigerator for 2-3 days and stirred from time to time in order to speed up crystallization, which occurs slowly. First crop of the product is filtered off and washed with a cold isopropanol-hexane 1:1 mixture (800 g), followed by 3x 500 cm³ of cold hexane. The product is dried at 40°C to yield 650 g of crystalline product with a purity of 98% (HPLC).

The filtrate is evaporated and the residue (1000 g), after addition of 500 g of isopropanol and 500 g of hexane, left in a refrigerator at +5°C then at -15°C, to obtain after several days of crystallization 300 g of the crystalline product (second crop), complying with the purity requirements (HPLC: 98%).

The residue from the second crop, after evaporation of solvents, in the form of brown oil (700 g), is dissolved in 5 dm³ of methylene chloride and after addition of 700 g of silica gel is stirred for 0.5 h. After filtration, the filtrate is evaporated. To 610 g of obtained light brown oil 500 g of isopropanol and 500 g of hexane are added and the mixture is allowed to crystallize in the refrigerator for 2 days. After filtering off, washing and drying 120 g of white crystalline product is obtained complying with the purity requirements (HPLC: 98%). 1070 g of final product is obtained in total with a purity above 98%. Yield 53.4%.
M.p. 111-119°C (isopropanol).
¹H NMR (400 MHz, CDCl₃) δ (ppm): 7.41-7.27 (m, 10 H), 5.32 (brd, 1H), 4.92 (dAB, 1H, *J* = 10.6 Hz), 4.89 (dAB, 1H, *J* = 10.6 Hz), 4.88 (dAB, 1H, *J* = 10.8 Hz), 4.60 (dAB, 1H, *J* = 10.8 Hz), 4.34 (dd, 1H, *J* = 2.2 Hz, *J*= 12.1 Hz), 4.22 (dd, *J* = 4.3 Hz, *J*= 12.1 Hz), 4.12 (ddd, *J* = 2.2 Hz, *J* = 4.3 Hz, *J*= 9.9 Hz), 4.05 (dd, 1H, *J*= 8.9 Hz, *J*= 10.1 Hz), 3.55 (dd, 1H, *J*= 8.9 Hz, *J*= 9.9 Hz), 3.44 (dd, 1H, *J*= 3.5 Hz, *J*= 10.1 Hz), 2.90, (brs, 1H), 2.04, (s, 3H).
¹³C NMR (100 MHz,CDCl₃) δ (ppm): 170.70, 137.61, 137.47, 128.57, 128.54, 128.15, 128.08, 128.03, 127,97, 92.09, 80.12, 77.96, 75.64, 75.12, 69.35, 64.02, 62.77, 20.85.

## Claims

1. A process for the preparation of 6-O-acetyl-2-azido-3,4-di-O-benzyl-2-deoxy-D-glucopyranose which comprises
a) reaction of 1,6-anhydro-2-azido-2-deoxy-D-glucopyranose with benzyl bromide in the presence of solid sodium hydroxide in acetonitrile to form 1,6-anhydro-2-azido-3,4-di-O-benzyl-2-deoxy-D-glucopyranose;
b) reaction of 1,6-anhydro-2-azido-3,4-di-O-benzyl-2-deoxy-D-glucopyranose with acetic anhydride in the presence of trifluoroacetic acid to form 1,6-di-O-acetyl-2-azido-3,4-di-O-benzyl-2-deoxy-D-glucopyranose; and
c) treating 1,6-di-O-acetyl-2-azido-3,4-di-O-benzyl-2-deoxy-D-glucopyranose with piperidine in THF to form 6-O-acetyl-2-azido-3,4-di-O-benzyl-2-deoxy-D-glucopyranose.

2. The process according to claim 1 **characterized in that** a product of step a) is isolated by filtering off inorganic residues, distilling off the solvent and crystallization from n-hexane.

3. The process according to claim 1 or 2 **characterized in that** the crude product from step c), after removal of piperidine, is treated by dissolving in isopropanol, addition of n-hexane to form a crystalline precipitate and filtering off the crystalline precipitate.

4. The process according to claim 3 **characterized in that** the filtrate from filtration of the crystalline precipitate is evaporated to remove organic solvents, and to the residue a mixture isopropanol/hexane is added to form a next crop of the crystalline precipitate of the product from step c), the crystalline precipitate is filtered off, and the crops of crystalline precipitate are combined.
